# EUROPÄISCHE PATENTSCHRIFT

(11) **EP 0 602 549 B1**
(45) Veröffentlichungstag und Bekanntmachung des Hinweises auf die Patenterteilung: **10.09.1997**
(21) Anmeldenummer: 93119885.7
(22) Anmeldetag: 09.12.1993
(51) Int. Cl.: C07C 65/03

(54) **4-Hydroxy-2,3,5-trifluorbenzoesäure und Verfahren zu ihrer Herstellung**
4-Hydroxy-2,3,5-trifluorobenzoic acid and a process for its preparation
Acide 4-hydroxy-2,3,5-trifluorbenzoique et son procédé de préparation

(30) Priorität: 17.12.1992 DE 4242696
(43) Veröffentlichungstag der Anmeldung: 22.06.1994
(73) Patentinhaber: HOECHST AKTIENGESELLSCHAFT, 65929 Frankfurt am Main (DE)
(72) Erfinder: Dr. Pfirmann, Ralf, D-64347 Griesheim (DE); Dr. Wingen, Rainer, D-65795 Hattersheim/Main (DE)

(56) Entgegenhaltungen:
- US-A- 3 413 341
- US-A- 3 459 794
- PATENT ABSTRACTS OF JAPAN vol. 10, no. 67 (C-333)(2124) 15. März 1986 & JP-A-60 204 742 (NIPPON SHOKUBAI KAGAKU KOGYO K.K.) 16. Oktober 1985

## Beschreibung

Die vorliegende Erfindung betrifft die neue Verbindung 4-Hydroxy-2,3,5-trifluorbenzoesäure und ein Verfahren zu ihrer Herstellung. Dieser neue Stoff läßt sich zur Herstellung neuer flüssigkristalliner Verbindungen mit vorteilhaften Eigenschaften verwenden. Die Synthese dieser flüssigkristallinen Verbindungen und deren Verwendung in Displays sowie ihre neuen vorteilhaften Eigenschaften sind Gegenstand einer separaten Anmeldung (Aktenzeichen P 4242695.2 ) mit dem Titel "Trifluorphenylverbindungen, Verfahren zu ihrer Herstellung und ihre Verwendung in flüssigkristallinen Mischungen". Diese Anmeldung besitzt die gleiche Priorität wie die vorliegende Erfindung. In dem dieser Anmeldung beigefügten Schema 5 ist der zu den flüssigkristallinen Verbindungen führende Reaktionsweg in vereinfachter Weise dargestellt. Lediglich aus Gründen der Verdeutlichung ist das entsprechende Schema in der vorliegenden Anmeldung ohne zusätzliche Erläuterungen wiedergegeben (vgl. Reaktionschema).

Zur Herstellung der 4-Hydroxy-2,3,5-trifluorbenzoesäure geht man zweckmäßigerweise von 2,3,4,5-Tetrafluorbenzoesäure aus. Dieses Ausgangsmaterial kann nach literaturbekannten Verfahren (DE 3 810 093; EP 0 218 111; EP 0 194 671) hergestellt werden. Man setzt 2,3,4,5-Tetrafluorbenzoesäure mit einer basischen Verbindung, die in Wasser gelöst und/oder suspendiert vorliegt, bei erhöhter Temperatur um. Man setzt gegebenenfalls ein fluoridfangendes Mittel zu, stellt das anfallende Reaktionsgemisch durch Zugabe einer Säure auf einen vorgegebenen pH-Wert ein und isoliert die gebildete 4-Hydroxy-2,3,5-trifluorbenzoesäure.

Die Umsetzung verläuft entsprechend dem nachfolgenden Formelschema, 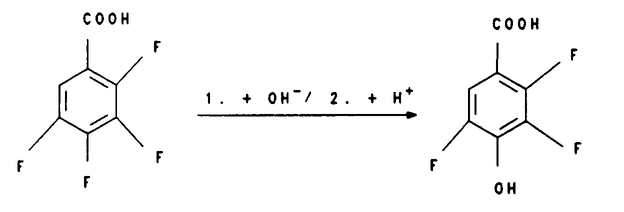 wobei das in 4-Position befindliche Fluoratom gegen eine Hydroxylgruppe ausgetauscht wird.

Üblicherweise löst man die basische Verbindung in Wasser und setzt diese wäßrige Lösung unmittelbar in die Reaktion ein. Es ist allerdings auch möglich, in den Fällen, in denen sich die basische Verbindung nicht im gewünschten Umfange in Wasser löst, eine Suspension der basischen Verbindung in Wasser zu verwenden. Zweckmäßigerweise setzt man die basische Verbindung in einer Konzentration von 3 bis 50, insbesondere 5 bis 40, bevorzugt 10 bis 25 Gew.-%, bezogen jeweils auf die wäßrige Lösung oder auf die wäßrige Suspension, ein.

Als basische Verbindungen eignen sich basische Verbindungen von Alkalimetallen und/oder Erdalkalimetallen. Hierunter fallen Alkalioxide, Alkalihydroxide, basische Alkalisalze, Erdalkalioxide, Erdalkalihydroxide und/oder basische Erdalkalisalze. Geeignete basische Verbindungen sind Lithiumhydroxid, Natriumhydroxid, Kaliumhydroxid, Rubidiumhydroxid, Cäsiumhydroxid, Lithiumhydrogencarbonat, Lithiumcarbonat, Natriumhydrogencarbonat, Natriumcarbonat, Kaliumhydrogencarbonat, Kaliumcarbonat, basisches Natriumphosphat, basisches Kaliumphosphat, Magnesiumhydroxid, Calciumhydroxid, Strontiumhydroxid und/oder Bariumhydroxid.

Gut geeignet als basische Verbindung sind Natriumhydroxid, Kaliumhydroxid und/oder Calciumhydroxid, insbesondere Natriumhydroxid und/oder Kaliumhydroxid, bevorzugt Natriumhydroxid. Mit gutem Erfolg lassen sich auch gemischte Salze, beispielsweise basische Carbonate und Phosphate oder auch Mischungen dieser basischen Verbindung verwenden.

Bei Durchführung der erfindungsgemäßen Umsetzung ist darauf zu achten, daß die basische Verbindung in einer ausreichenden Menge eingesetzt wird, um die gewünschte Substitution des in 4-Stellung befindlichen Fluoratoms herbeizuführen. Üblicherweise setzt man die basische Verbindung in einer solchen Menge ein, daß je Mol 2,3,4,5-Tetrafluorbenzoesäure 3 bis 10, insbesondere 3,1 bis 4,8, bevorzugt 3,3 bis 4,2 Mol Hydroxidionen zu Verfügung stehen.

Obgleich die Umsetzung der 2,3,4,5-Tetrafluorbenzoesäure bereits bei vergleichsweise niedrigen Temperaturen abläuft, empfiehlt es sich, die Umsetzung bei erhöhter Temperatur vorzunehmen. Üblicherweise läßt sich die Umsetzung bei 50 bis 180 °C mit gutem Erfolg durchführen. In den meisten Fällen genügt es, die Umsetzung bei einer Temperatur von 70 bis 130, insbesondere 90 bis 110 °C durchzuführen. Die zu wählende Reaktionstemperatur hängt unter anderem sowohl von den Mengen der einzusetzenden Stoffe als auch von der Art und der Konzentration der basischen Verbindung ab. Daneben spielen auch die Größe des Reaktionsansatzes und die Art der verwendeten Reaktionsapparatur in begrenztem Umfange eine Rolle. Höhere Reaktionstemperaturen, insbesondere oberhalb 100 °C, erfordern in der Regel eine Arbeitsweise unter Druck. Das bedeutet, daß die verwendete Reaktionsapparatur für eine derartige Arbeitsweise ausgelegt sein muß. Hingegen ermöglichen Temperaturen unterhalb 100 °C üblicherweise eine Reaktionsdurchführung unter Normaldruck.

Ein besonderer Vorteil des erfindungsgemäßen Verfahrens besteht darin, daß man die Umsetzung in Abwesenheit eines zusätzlichen Lösungsmittels, insbesondere eines organischen Lösungsmittels durchführen kann. In Hinblick darauf, daß organische Solventien als Lösungsvermittler fungieren, ist es als überraschend anzusehen, daß man bei der Umsetzung auf Zusatz eines derartigen Lösungsmittels verzichten kann und trotzdem die gewünschte Verbindung in hoher Ausbeute und hoher Reinheit erhält. Nebenprodukte, insbesondere die isomere 3-Hydroxy-2,4,5-trifluorbenzoesäure, werden überraschenderweise nur in vergleichsweise geringem Umfange gebildet. Dieses Nebenprodukt tritt üblicherweise in einer Menge weniger als 6 Mol-% auf. Insbesondere überraschend ist neben der hohen Selektivität, daß die Umsetzung bei den erfindungsgemäßen Temperaturen abläuft, da die Carboxylat-Gruppierung eine auf diesen Reaktionstyp eher desaktivierende Wirkung ausübt. Aus demselben Grunde wäre ein Eintritt der Hydroxy-Gruppe in 3- oder 5-Position zu erwarten, da hierbei im Übergangszustand keine störenden Wechselwirkungen zweier negativer Ladungen auftreten sollten. Es ist daher als besonders überraschend zu werten, daß von 4 möglichen Fluoratomen nur eines mit hoher Selektivität ausgetauscht wird und auch der Austausch eines zweiten Fluoratoms in nur untergeordnetem Maße eintritt.

Bei der Aufarbeitung des anfallenden Reaktionsgemisches besteht die Gefahr, daß die verwendeten Reaktionsapparaturen durch Korrosion, hervorgerufen durch die Bildung von Fluorwasserstoff, beschädigt werden. Diese Korrosion läßt sich durch Zusatz von fluoridfangenden Mitteln gering halten und überraschenderweise weitestgehend unterdrücken. Geeignete fluoridfangende Mittel sind Calciumsalze und/oder Siliciumverbindungen. Als Calciumsalze lassen sich Calciumchlorid, Calciumsulfat und/oder Calciumhydroxid und als Siliciumverbindungen verschiedene Silikate, Kieselgele und/oder Siliciumdioxid, insbesondere Siliciumdioxid mit erhöhter innerer Oberfläche verwenden. Die fluoridfangenden Mittel werden üblicherweise in der 0,5 bis 10-fachen, insbesondere 1,5 bis 4-fachen Menge, bezogen auf die theoretisch entstandene Fluoridmenge, eingesetzt. Es ist überraschend, daß sich die durch Fluorwasserstoffsäure verursachte Korrosion auch in der bei dem erfindungsgemäßen Verfahren entstehenden verdünnten wäßrigen Lösung weitgehend unterdrücken läßt.

Als Folge der Umsetzung liegt die 4-Hydroxy-2,3,5-trifluorbenzoesäure nicht in Form der freien Säure, sondern als eine wäßrige Lösung eines Salzes vor. Um die freie 4-Hydroxy-2,3,5-trifluorbenzoesäure aus der wäßrigen Lösung ihrer Salze freizusetzen, säuert man das Reaktionsgemisch an. Als Säure kann eine Mineralsäure, insbesondere eine nicht oxidierende Mineralsäure, oder eine organische Säure ausreichender Säurestärke verwendet werden. Als Mineralsäure eignet sich Schwefelsäure, Phosphorsäure, Salzsäure, Bromwasserstoffsäure und/oder Jodwasserstoffsäure und als organische Säure Ameisensäure, eine gegebenenfalls halogenierte Essigsäure, eine aromatische und/oder eine aliphatische Sulfonsäure oder Mischungen dieser Säuren.

Üblicherweise setzt man soviel Säure zu, daß ein pH-Wert von 0 bis 6 eingestellt wird. In zahlreichen Fällen erweist es sich als günstig, einen pH-Wert von 0,1 bis 4, insbesondere 1 bis 3 einzustellen.

Die durch Ansäuern freigesetzte 4-Hydroxy-2,3,5-trifluorbenzoesäure wird aus dem anfallenden Reaktionsgemisch isoliert, indem man das Reaktionsgemisch mit einem geeigneten Lösungsmittel extrahiert. Hierfür kommen Ether, insbesondere aliphatische Ether, beispielsweise MTBE (Methyl-tert.-butylether), Di-n-butylether, und/oder Ester, insbesondere Ester aliphatischer Carbonsäuren, bevorzugt Essigsäurealkylester oder Essigsäurealkoxyalkylester, beispielsweise Essigsäuremethylester, Essigsäureethylester, Essigsäurebutylester oder Essigsäure-3-methoxy-butylester in Betracht. Man destilliert das Lösungsmittel ab und kristallisiert das erhaltene Wertprodukt in einem geeigneten Lösungsmittel, beispielsweise 1,2-Dichlorbenzol, Chlorbenzol, Toluol, Xylol, Chlortoluol, Cyclohexan, Methylcyclohexan, um. Ein anderer Weg besteht darin, das Wasser aus dem nach Ansäuern anfallenden Reaktionsgemisch mittels eines organischen Lösungsmittels, beispielsweise Cyclohexan, Toluol, Xylol, Chlorbenzol oder 1,2-Dichlorbenzol, azeotrop abzudestillieren, die heiße Suspension zu filtrieren, wobei vorhandene Salze abgetrennt werden, und das Wertprodukt unter Erkalten der wäßrigen Lösung auszukristallisieren. Die weitere Reinigung erfolgt, wie zuvor angegeben, durch Umkristallisieren in einem hierfür geeigneten Lösungsmittel.

Das nachfolgende Beispiel belegt die Erfindung, ohne sie zu beschränken.

### Beispiel

50 g (0.258 Mol) 2,3,4,5-Tetrafluorbenzoesäure und 41,4 g (1,04 Mol) Natriumhydroxid werden in 687 g Wasser vorgelegt, die entstehende Lösung wird auf 100 °C geheizt und 12 bis 14,5 Stunden bei dieser Temperatur gerührt. Der Endpunkt der Umsetzung wird gaschromatographisch bestimmt. Anschließend werden 57,2 g Calciumchlorid zugegeben und es wird mit 30 %iger Salzsäure auf pH 1 eingestellt. Die Lösung wird kontinuierlich 16 Stunden mit MTBE (Methyl-tert.-butylether) extrahiert und danach wird das Lösungsmittel abdestilliert. Der trockene Rückstand (44,6 g) wird bei 145 °C aus 1,2-Dichlorbenzol umkristallisiert. Man erhält 42,1 g (0,219 Mol, 85 %) weiße bis leicht beige gefärbte 4-Hydroxy-2,3,5-trifluorbenzoesäure, die bei sämtlichen analytischen Untersuchungen die erwarteten Werte liefert. Der bei der Umsetzung mitentstandene Anteil an 3-Hydroxy-2,4,5-trifluorbenzoesäure von weniger als 5 Mol-% des Rohproduktes wird durch die Umlösung (Umkristallisation) vollständig entfernt.

Alternativ läßt sich das Produkt isolieren, indem man das Wasser aus der Mutterlauge mittels Xylol oder 1,2-Dichlorbenzol azeotrop abdestilliert, die erhaltene Suspension heiß von den ungelösten Salzen abfiltriert und anschließend das Produkt auskristallisieren läßt. Die erforderliche weitere Reingung erfolgt, wie zuvor beschrieben, durch erneute Kristallisation aus 1,2-Dichlorbenzol oder Xylol.

Setzt man anstatt Natriumhydroxid Kaliumhydroxid (56,1 g, 1 Mol) ein und säuert man mit Salzsäure an und verwendet man Calciumhydroxid (30 g) an Stelle von Calciumchlorid als fluoridfangendes Mittel, so erhält man im wesentlichen dasselbe Ergebnis.

Anstatt Natriumhydroxid kann Lithiumcarbonat (88,7 g), Lithiumhydroxid-Hydrat (37,8 g) oder Calciumhydroxid (148,2 g) verwendet werden, wobei dasselbe Ergebnis erhalten wird. Verwendet man Schwefelsäure oder Phosphorsäure statt Salzsäure zum Ansäuern der Reaktionsmischung, so erhält man im wesentlichen dasselbe Ergebnis.
Schmp. (DSC): 164,1 °C
¹H-NMR [DMSO-d₆/ppm]:
   - δ =: 7,46 (q, 1H, J_{AD} = 11,3 Hz, J_{BD} = 6,6 Hz, J_{CD} = 2,4 Hz, Ar-H⁶ (D))
¹⁹F-NMR [DMSO-d₆/ppm]:
   - δ =: -136,26 (q, 1F, J_{AD} = 11,3 Hz, J_{AB} = 12,7 Hz, J_{AC} = 9,4 Hz, Ar-F⁵ (A))
   -138,6 (q, 1F, J_{BD} = 6,6 Hz, J_{AB} = 12,7 Hz, J_{BC} = 21,0 Hz, Ar-F² (B))
   -154,94 (q, 1F, J_{CD} = 2,4 Hz, J_{BC} = 21,0 Hz, J_{AC} = 9,4 Hz, AR-F³ (C))

| | | | | |
|---|---|---|---|---|
| C₇H₃F₃O₃ (192.094) | Gew.-% berechnet | C 43,77 | H 1,57 | F 29,67 |
| | Gew.% gefunden | C 43,8 | H 1,6 | F 29,6 |

- IR (KBr) [cm⁻¹]:: 3660, 3340, 3600-2200 (br), 1690, 1630, 1530, 1490, 1410, 1330, 1300,1250, 1200, 1100, 1010, 905, 785, 740, 715, 480, 460
- MS (m/z) [%]:: 45 (5,2), 69 (9,5), 75 (6,1), 80 (5,9), 99 (18,3), 119 (26,5), 146 (3,8), 147 (14,1), 175 (100), 176 (9,0), 192 (69,9, M⁺), 193 (6,1)

Das nachfolgende Reaktionsschema dient lediglich zur Verdeutlichung der Herstellung flüssigkristalliner Verbindungen unter Verwendung von 4-Hydroxy-2,3,5-trifluorbenzoesäure.

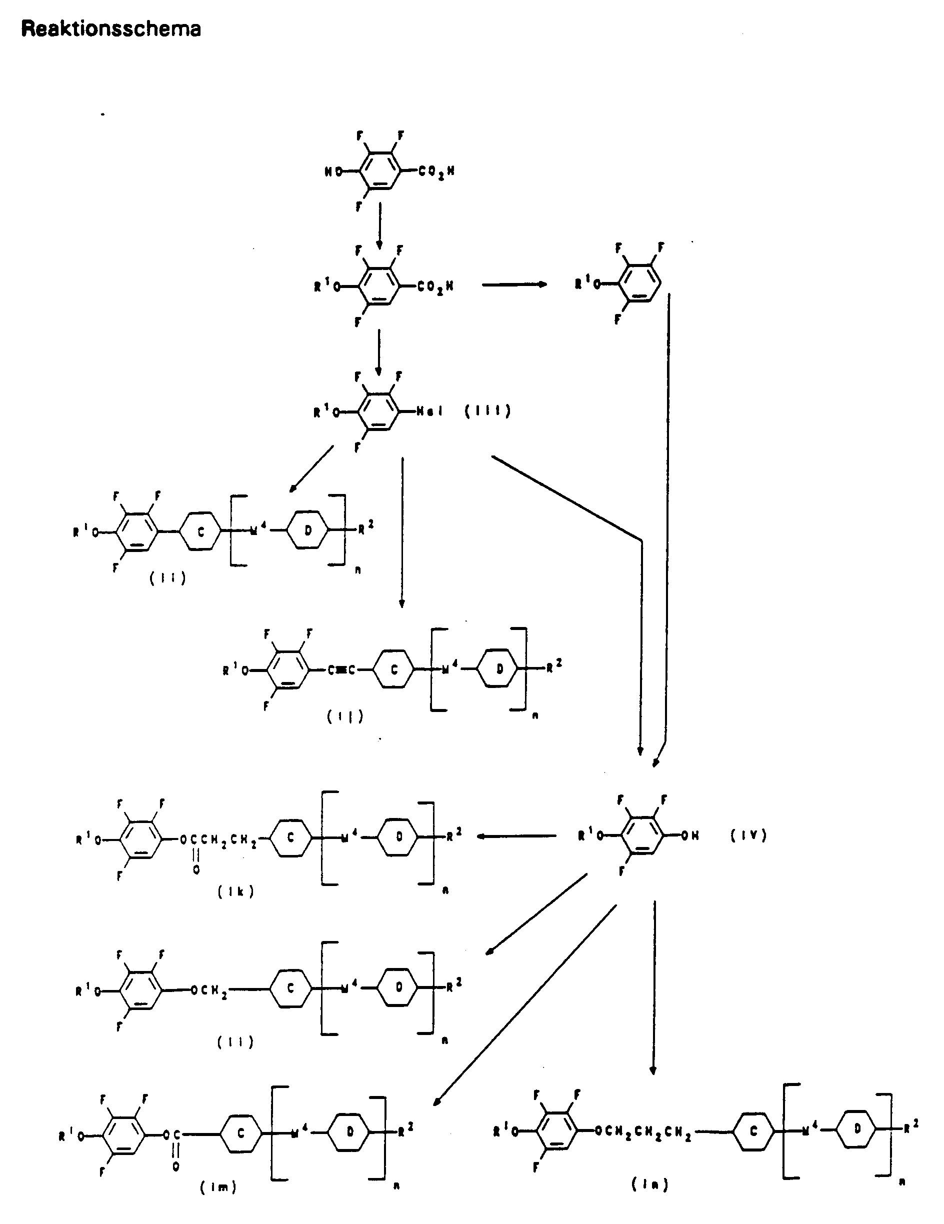

## Patentansprüche

1. 4-Hydroxy-2,3,5-trifluorbenzoesäure der Formel

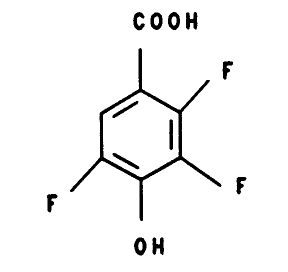

2. Verfahren zur Herstellung von 4-Hydroxy-2,3,5-trifluorbenzoesäure, dadurch gekennzeichnet, daß man 2,3,4,5-Tetrafluorbenzoesäure mit einer in Wasser gelösten und/oder suspendierten basischen Verbindung bei erhöhter Temperatur umsetzt, gegebenenfalls ein fluoridfangendes Mittel zusetzt, das anfallende Reaktionsgemisch durch Zugabe einer Säure auf einen pH-Wert von 0 bis 6 einstellt und die gebildete 4-Hydroxy-2,3,5-trifluorbenzoesäure isoliert.

3. Verfahren nach Anspruch 2, dadurch gekennzeichnet, daß man die basische Verbindung in einer Konzentration von 3 bis 50 insbesondere 5 bis 40, bevorzugt 10 bis 25 Gew.-%, bezogen auf die wäßrige Lösung oder Suspension, einsetzt.

4. Verfahren nach Anspruch 2 oder 3, dadurch gekennzeichnet, daß man als basische Verbindung ein Alkalioxid, Alkalihydroxid, mein basisches Alkalisalz, ein Erdalkalioxid, Erdalkalihydroxid und/oder ein basisches Erdalkalisalz einsetzt.

5. Verfahren nach einem oder mehreren der Ansprüche 2 bis 4, dadurch gekennzeichnet, daß man als basische Verbindung Lithiumhydroxid, Natriumhydroxid, Kaliumhydroxid, Rubidiumhydroxid, Cäsiumhydroxid, Natriumhydrogencarbonat, Natriumcarbonat, Kaliumhydrogencarbonat, Kaliumcarbonat, ein basisches Natriumphosphat, ein basisches Kaliumphosphat, Magnesiumhydroxid, Calciumhydroxid, Strontiumhydroxid und/oder Bariumhydroxid umsetzt.

6. Verfahren nach einem oder mehreren der Ansprüche 2 bis 5, dadurch gekennzeichnet, daß man als basische Verbindung Natriumhydroxid, Kaliumhydroxid und/oder Calciumhydroxid, insbesondere Natriumhydroxid und/oder Kaliumhyroxid, bevorzugt Natriumhydroxid einsetzt.

7. Verfahren nach einem oder mehreren der Ansprüche 2 bis 6, dadurch gekennzeichnet, daß man die basische Verbindung in einer solchen Menge einsetzt, daß je Mol 2,3,4,5-Tetrafluorbenzoesäure 3 bis 10, insbesondere 3,1 bis 4,8, bevorzugt 3,3 bis 4,2 Mol Hydroxidionen zur Verfügung stehen.

8. Verfahren nach einem oder mehreren der Ansprüche 2 bis 7, dadurch gekennzeichnet, daß man die Umsetzung bei 50 bis 180, insbesondere 70 bis 130, bevorzugt 90 bis 110 °C durchführt.

9. Verfahren nach einem oder mehreren der Ansprüche 2 bis 8, dadurch gekennzeichnet, daß man die Umsetzung in Abwesenheit eines zusätzlichen Lösungsmittels, insbesondere eines organischen Lösungsmittels durchführt.

10. Verfahren nach einem oder mehreren der Ansprüche 2 bis 9, dadurch gekennzeichnet, daß man als fluoridfangendes Mittel ein Calciumsalz, insbesondere Calciumchlorid, Calciumsulfat und/oder Calciumhydroxid und/oder eine Siliciumverbindung, insbesondere ein Silikat, ein Kieselgel und/oder Silicumdioxid, bevorzugt ein Siliciumdioxid mit erhöhter innerer Oberfläche einsetzt.

11. Verfahren nach einem oder mehreren der Ansprüche 2 bis 10, dadurch gekennzeichnet, das als Säure eine Mineralsäure, insbesondere eine nichtoxidierende Mineralsäure, oder eine organische Säure ausreichender Säurestärke einsetzt.

12. Verfahren nach einem oder mehreren der Ansprüche 2 bis 11, dadurch gekennzeichnet, daß man als Säure Schwefelsäure, Phosphorsäure, Salzsäure, Bromwasserstoffsäure, Jodwasserstoffsäure, Ameisensäure, eine gegebenenfalls halogenierte Essigsäure, eine aliphatische oder aromatische Sulfonsäure (oder Mischungen dieser Säuren) einsetzt.

13. Verfahren nach einem oder mehreren der Ansprüche 2 bis 12, dadurch gekennzeichnet, daß man das Reaktionsgemisch durch Zugabe von Säure auf einen pH-Wert von 0,1 bis 4, insbesondere 1 bis 3 einstellt.

## Claims

1. 4-Hydroxy-2,3,5-trifluorobenzoic acid, which has the formula

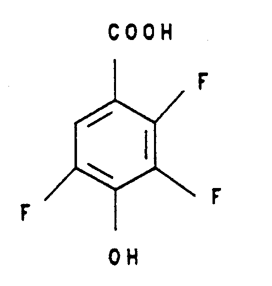

2. A process for preparing 4-hydroxy-2,3,5-trifluorobenzoic acid, which comprises reacting 2,3,4,5-tetrafluorobenzoic acid at elevated temperature with a basic compound dissolved and/or suspended in water, adding, if desired, a fluoride scavenger, bringing the resulting reaction mixture to a pH of 0 to 6 by addition of an acid, and isolating the 4-hydroxy-2,3,5-trifluorobenzoic acid formed.

3. The process as claimed in claim 2, wherein the basic compound is used in a concentration of 3 to 50, in particular 5 to 40, preferably 10 to 25, % by weight, based on the aqueous solution or suspension.

4. The process as claimed in claim 2 or 3, wherein the basic compound used is an alkali metal oxide, alkali metal hydroxide, a basic alkali metal salt, an alkaline earth metal oxide, alkaline earth metal hydroxide and/or a basic alkaline earth metal salt.

5. The process as claimed in one or more of claims 2 to 4, wherein the basic compound reacted is lithium hydroxide, sodium hydroxide, potassium hydroxide, rubidium hydroxide, cesium hydroxide, sodium bicarbonate, sodium carbonate, potassium bicarbonate, potassium carbonate, a basic sodium phosphate, a basic potassium phosphate, magnesium hydroxide, calcium hydroxide, strontium hydroxide and/or barium hydroxide.

6. The process as claimed in one or more of claims 2 to 5, wherein the basic compound used is sodium hydroxide, potassium hydroxide and/or calcium hydroxide, in particular sodium hydroxide and/or potassium hydroxide, preferably sodium hydroxide.

7. The process as claimed in one or more of claims 2 to 6, wherein the basic compound is used in such an amount that 3 to 10, in particular 3.1 to 4.8, preferably 3.3 to 4.2, mol of hydroxide ions are available per mole of 2,3,4,5-tetrafluorobenzoic acid.

8. The process as claimed in one or more of claims 2 to 7, wherein the reaction is carried out at 50 to 180, in particular 70 to 130, preferably 90 to 110, °C.

9. The process as claimed in one or more of claims 2 to 8, wherein the reaction is carried out in the absence of an additional solvent, in particular an organic solvent.

10. The process as claimed in one or more of claims 2 to 9, wherein the fluoride scavenger used is a calcium salt, in particular calcium chloride, calcium sulfate and/or calcium hydroxide, and/or a silicon compound, in particular a silicate, a silica gel and/or silicon dioxide, preferably a silicon dioxide having increased inner surface area.

11. The process as claimed in one or more of claims 2 to 10, wherein the acid used is a mineral acid, in particular a non-oxidizing mineral acid, or an organic acid of sufficient acid strength.

12. The process as claimed in one or more of claims 2 to 11, wherein the acid used is sulfuric acid, phosphoric acid, hydrochloric acid, hydrobromic acid, hydriodic acid, formic acid, a halogenated or non-halogenated acetic acid, an aliphatic or aromatic sulfonic acid (or mixtures of these acids).

13. The process as claimed in one or more of claims 2 to 12, wherein the reaction mixture is brought to a pH of 0.1 to 4, in particular 1 to 3, by addition of acid.

## Revendications

1. Acide 4-hydroxy-2,3,5-trifluorobenzoïque de formule

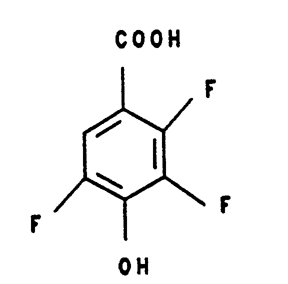

2. Procédé pour préparer l'acide 4-hydroxy-2,3,5-trifluorobenzoïque, caractérisé en ce qu'on fait réagir à haute température de l'acide 2,3,4,5-tétrafluorobenzoïque avec un composé basique en solution et/ou en suspension dans l'eau, on ajoute éventuellement un agent fixateur de fluorure, on ajuste à un pH de 0 à 6, par addition d'un acide, le mélange réactionnel qui se forme, et on isole l'acide 4-hydroxy-2,3,5-trifluorobenzoïque formé.

3. Procédé selon la revendication 2, caractérisé en ce qu'on utilise le composé basique à une concentration de 3 à 50, en particulier de 5 à 40 et de préférence de 10 à 25 % en poids par rapport à la solution ou à la suspension aqueuse.

4. Procédé selon la revendication 2 ou 3, caractérisé en ce qu'on utilise en tant que composé basique un oxyde d'un métal alcalin, un hydroxyde d'un métal alcalin, un sel basique d'un métal alcalin, un oxyde d'un métal alcalino-terreux, un hydroxyde d'un métal alcalinoterreux et/ou un sel basique d'un métal alcalino-terreux.

5. Procédé selon l'une ou plusieurs des revendications 2 à 4, caractérisé en ce qu'on fait réagir en tant que composé basique l'hydroxyde de lithium, l'hydroxyde de sodium, l'hydroxyde de potassium, l'hydroxyde de rubidium, l'hydroxyde de sodium l'hydrogénocarbonate de potassium, le carbonate de potassium, l'hydrogénocarbonate de sodium, le carbonate de sodium, l'hydrogénocarbonate de potassium, le carbonate de potassium, un phosphate basique de sodium, un phosphate basique de potassium, l'hydroxyde de magnésium, l'hydroxyde de calcium, l'hydroxyde de strontium et/ou l'hydroxyde de baryum.

6. Procédé selon l'une ou plusieurs des revendications 2 à 5, caractérisé en ce qu'on utilise en tant que composé basique l'hydroxyde de sodium, l'hydroxyde de potassium et/ou l'hydroxyde de calcium, en particulier l'hydroxyde de sodium et/ou l'hydroxyde de potassium, de préférence l'hydroxyde de potassium.

7. Procédé selon l'une ou plusieurs des revendications 2 à 6, caractérisé en ce qu'on utilise le composé basique en une quantité telle que l'on dispose par mode d'acide 2,3,4,5-tétrafluorobenzoïque de 3 à 10, en particulier de 3,1 à 4,8, de préférence de 3,3 à 4,2 modes d'ions hydroxyde.

8. Procédé selon l'une ou plusieurs des revendications 2 à 7, caractérisé en ce qu'on met en oeuvre la réaction à une température de 50 à 180°C, en particulier de 70 à 130°C, de préférence de 90 à 110°C.

9. Procédé selon l'une ou plusieurs des revendications 2 à 8, caractérisé en ce qu'on met en oeuvre la réaction en l'absence d'un solvant additionnel, en particulier d'un solvant organique.

10. Procédé selon l'une ou plusieurs des revendications 2 à 9, caractérisé en ce qu'on utilise en tant qu'agent fixateur de fluorure un sel de cadcium, en particulier le chlorure de calcium, le sulfate de calcium et/ou l'hydroxyde de cadcium, et/ou un composé du silicium, en particulier un silicate, un gel de sidice et/ou du dioxyde de sidicium, de préférence un dioxyde de silicium ayant une grande aire interne.

11. Procédé selon l'une ou plusieurs des revendications 2 à 10, caractérisé en ce qu'on utilise en tant qu'acide un acide minéral, en particulier un acide minéral non-oxydant, ou un acide organique suffisamment fort.

12. Procédé selon l'une ou plusieurs des revendications 2 à 11, caractérisé en ce qu'on utilise en tant qu'acide l'acide sulfurique, l'acide phosphorique, l'acide chlorhydrique, l'acide bromhydrique, l'acide iodhydrique, l'acide formique, un acide acétique éventuellement halogéné, un acide sulfonique aliphatique ou aromatique (ou des mélanges de ces acides).

13. Procédé selon l'une ou plusieurs des revendications 2 à 12, caractérisé en ce que, par addition d'un acide, on ajuste le mélange réactionnel à un pH de 0,1 à 4, en particulier de 1 à 3.
